# EUROPEAN PATENT SPECIFICATION

(11) **EP 3 508 268 B1**
(45) Date of publication and mention of the grant of the patent: **25.10.2023**
(21) Application number: 16915137.0
(22) Date of filing: 31.08.2016
(51) Int. Cl.: A23J 7/00, A61K 8/55, A61K 9/107, A61K 47/24, C07F 9/10, A23D 7/01

(54) **EGG YOLK PHOSPHOLIPID COMPOSITION AND FAT EMULSION AND LIPOLYSIS FORMULATION USING EGG YOLK PHOSPHOLIPID COMPOSITION**
EIGELBPHOSPHOLIPIDZUSAMMENSETZUNG SOWIE FETTEMULSION UND LIPOLYSEFORMULIERUNG UNTER VERWENDUNG DER EIGELBPHOSPHOLIPIDZUSAMMENSETZUNG
COMPOSITION DE PHOSPHOLIPIDE DE JAUNE D' OEUF, ET FORMULATION DE LIPOLYSE ET D'ÉMULSION DE GRAISSE À L'AIDE D'UNE COMPOSITION DE PHOSPHOLIPIDE DE JAUNE D' OEUF

(43) Date of publication of application: 10.07.2019
(73) Proprietor: Kewpie Corporation, Tokyo 150-0002 (JP)
(72) Inventor: AMANO, Yohei, Chofu-shi Tokyo 182-0002 (JP); SHONO, Junichi, Chofu-shi Tokyo 182-0002 (JP)
(74) Representative: Winter, Brandl - Partnerschaft mbB
(86) International application number: PCT/JP2016/075562
(87) International publication number: WO 2018/042576

(56) References cited:
- EP-A1- 2 695 527
- WO-A1-98/21215
- WO-A1-2011/061995
- CN-C- 1 270 720
- JP-A- S6 239 593
- JP-A- H09 224 584
- JP-A- S54 120 607
- JP-A- S54 126 206
- JP-A- S62 281 884
- JP-A- 2005 290 308
- US-A- 3 873 720
- US-A- 4 847 015
- US-A1- 2014 112 978
- AMARI J V ET AL: "ISOLATION AND PURIFICATION OF LECITHIN BY PREPARATIVE HIGH- PERFORMANCE LIQUID CHROMATOGRAPHY", JOURNAL OF CHROMATOGRAPHY A, ELSEVIER, AMSTERDAM, NL, vol. 517, no. 26, 1 September 1990 (1990-09-01), pages 219-228, XP000163030, ISSN: 0021-9673, DOI: 10.1016/S0021-9673(01)95723-1
- HIDETOSHI SUGINO et al.: "Ran'o Abura-Ran'o Lecithin no Tokusei to Oyo", Food Processing and Ingredients, vol. 33, no. 1, January 1998 (1998-01), pages 46-50, XP009514947,
- HARUYOSHI KAWAGUCHI et al.: "Shihan Keiran no Eiyo Seibun ni Tsuite - Nutritive Components in Commercial Chicken Egg", Report of Toyo College of Food Technology and Toyo Institute of Food Technology, vol. 19 1992, pages 61-68, XP009515866, Retrieved from the Internet: URL:http://www. shokuken.or.jp/report/19/19008.html

## Description

### Technical Field

The present invention relates to an egg yolk phospholipid composition, and a fat emulsion and a lipoid preparation produced using the egg yolk phospholipid composition.

### Background Art

Egg yolk phospholipids are widely used as, for example, emulsifying agents for fat emulsions, lipoid preparation, and the like in the fields of cosmetics and pharmaceutical products (WO 2009 / 093 650 A). US 4 847 015 A1 relates to a process for producing egg yolk lecithin having reduced phosphatidylethanolamine (PE) content. EP 2 695 527 A1 discloses an aerated oil-in-water emulsion composition comprising water, edible oil, egg yolk fraction and hydrophobin, having a pH ranging from 3 to 6. US 3 873 720 A1 relates to a fat emulsion, which contains at least three major nutrients, i.e. fat, carbohydrate and amino acids, and a method preparing same. US 2014 0 112 978 A1 relates to an ibuprofen-based compound, a method for preparation of the compound, and use of the compound in preparation of non-steroidal anti-inflammatory drugs. In recent years, there has been a demand for a high-purity emulsifying agent having excellent stability, which uses egg yolk phospholipids.

### Summary of Invention

### Technical Problem

The present invention provides an egg yolk phospholipid composition used for the production of a high-purity emulsion having excellent stability, and a fat emulsion and a lipoid preparation produced using the egg yolk phospholipid composition.

### Solution to Problem

This object is solved by the subject matter of the independent claims. Further aspects are disclosed in the subclaims. The inventors of the present invention found that a high-purity emulsion having excellent stability can be produced by adjusting the content of lysophosphatidylethanolamine and the content of amino acids in egg yolk phospholipids to predetermined amounts, thus completing the present invention.

### Advantageous Effects of Invention

The egg yolk phospholipid composition has a content of lysophosphatidylethanolamine of 1% by mass or less and a content of amino acids of from 50 mg/100 g to 500 mg/100 g. Thus, since the amount of amino acids in the egg yolk phospholipids is adjusted to the above-mentioned range, a high-purity emulsion having excellent stability can be produced using the egg yolk phospholipid composition.

The fat emulsion consists of emulsified particles containing the egg yolk phospholipid composition; and an aqueous phase in which the emulsified particles are dispersed. Therefore, the fat emulsion has high purity and has excellent emulsion stability.

The lipoid preparation consists of emulsified particles containing the egg yolk phospholipid composition and a drug; and an aqueous phase in which the emulsified particles are dispersed. Therefore, the lipoid preparation has high purity and excellent emulsion stability.

### Description of Embodiments

In the following description, the present invention will be explained in detail with reference to the drawings. According to the invention, unless particularly stated otherwise, the unit "parts" means "parts by mass", and the unit "percent (%)" means "percent (%) by mass".

An egg yolk phospholipid composition according to an embodiment of the invention has a content of lysophosphatidylethanolamine (hereinafter, also referred to as "LPE") of 1% by mass or less and a content of amino acids of from 50 mg/100 g to 500 mg/100 g.

When the egg yolk phospholipid composition according to the present embodiment is used, since the content of LPE Is 1% by mass or less, and the content of amino acids is from 50 mg/100 g to 500 mg/100 g, an emulsion having high purity and excellent stability can be produced by using the egg yolk phospholipid composition.

The inventors of the present invention found that a decrease in pH that occurs at the time of a high temperature treatment intended for sterilizing an emulsion obtainable using the egg yolk phospholipid composition according to the present embodiment, is attributed to the content of amino acids that are eliminated together with LPE at the time of LPE elimination. A decrease in pH is one of the factors that decrease the stability of the emulsion.

More specifically, in the egg yolk phospholipid composition according to the present embodiment, since the content of LPE is suppressed to a level of 1% by mass or less, and the content of amino acids is adjusted to be from 50 mg/100 g to 500 mg/100 g, an emulsion that uses the egg yolk phospholipid composition according to the present embodiment has high purity, and even if the emulsion is subjected to a high temperature treatment at the time of sterilization, the emulsion undergoes a reduced decrease in pH and has excellent stability.

### (Egg yolk phospholipid composition)

According to the invention, the term "egg yolk phospholipid composition" means that the composition includes egg yolk-derived phospholipids.

### (Phospholipids)

Example of the phospholipids include phosphatidylcholine (hereinafter, also referred to as "PC"), phosphatidylethanolamine (hereinafter, referred to as "PE"), phosphatidylinositol, phosphatidylserine, phosphatidylglycerol, phosphatidyl ethylene glycol, phosphatidyl polyethylene glycol, phosphatidic acid, lysophosphatidylcholine, lysophosphatidylethanolamine, lysophosphatidylglycerol, lyso-forms of the above-mentioned phospholipids, and sphingomyelin. Among these, the phospholipids can include two or more kinds thereof. These are all egg yolk-derived phospholipids.

The content (total amount) of egg yolk phospholipids in the egg yolk phospholipid composition according to the present embodiment is 65% by mass or more and less than 100% by mass, and the content is usually 99.95% by mass or less. From the viewpoint that an emulsion having excellent stability can be produced, the content of egg yolk phospholipids is preferably 75% by mass or more, and is 99.5% by mass or less, and more preferably 95% by mass or less.

Above all, the egg yolk phospholipid composition according to the present embodiment includes PC at a proportion of 65% by mass or more (preferably 68% by mass or more, more preferably 70% by mass or more, and preferably 99% by mass or less, more preferably 95% by mass or less, and even more preferably 90% by mass or less), and includes PE at a proportion of 5% by mass or more (preferably 7% by mass or more, more preferably 10% by mass or more, and preferably 20% by mass or less).

The content of LPE included in the egg yolk phospholipid composition according to the present embodiment is 1% by mass or less, and may be 0.8% by mass or less. When the content of LPE is less than or equal to the upper limit described above, an egg yolk phospholipid composition having high purity and high stability can be obtained. The lower limit of the content of LPE is not particularly limited; however, when the content is 0.01% by mass or more, and 0.1% by mass or more, an egg yolk phospholipid composition having high stability may be easily obtained.

Furthermore, from the viewpoint of having superior stability, the egg yolk phospholipid composition according to the present embodiment includes PE, and in a case in which the content of this PE is designated as 100 parts by mass, the content of the LPE with respect to the content of the PE is preferably from 2 parts by mass to 15 parts by mass, more preferably 3 parts by mass or more, and more preferably 10 parts by mass or less.

### (Amino acids)

In a case in which the egg yolk phospholipid composition according to the present embodiment includes two or more kinds of amino acids, the content of amino acids is the total amount of the two or more kinds of amino acids. In regard to the egg yolk phospholipid composition according to the present embodiment, from the viewpoint that stability can be further increased, the content (total amount) of the amino acids is preferably 50 mg/100 g or more, and from the viewpoint that purity of the phospholipid composition can be further increased, the content (total amount) of the amino acids is more preferably 500 mg/100 g or less, while the content is preferably 100 mg/100 g or less, and more preferably 400 mg/100 g or less.

Examples of the amino acids include essential amino acids (tryptophan, lysine, methionine, phenylalanine, threonine, valine, leucine, isoleucine, and histidine), non-essential amino acids (arginine, cysteine, tyrosine, glycine, alanine, serine, cysteine, asparagine, glutamine, proline, aspartic acid, and glutamic acid), and amino acids other than those described above. Among them, from the viewpoint that an emulsion having superior stability can be produced, it is preferable that the egg yolk phospholipid composition includes at least one selected from serine, threonine, lysine, and arginine, and it is more preferable that the egg yolk phospholipid composition includes serine and/or threonine.

In regard to the egg yolk phospholipid composition according to the present embodiment, from the viewpoint that an emulsion having superior stability can be produced, in a case in which the total amount of the amino acids is designated as 100 parts by mass, the proportion of the sum of serine and threonine in the amino acids is more preferably 10 parts by mass or more, and even more preferably 20 parts by mass or more. Meanwhile, the upper limit of the proportion of the sum of serine and threonine in the amino acids is not particularly limited; however, for example, from the viewpoint that it is difficult to obtain effects appropriate for the content, the upper limit can be set to be 50 parts by mass or less.

Furthermore, in regard to the egg yolk phospholipid composition according to the present embodiment, from the viewpoint that an emulsion having excellent stability can be produced, in a case in which the total amount of the amino acids is designated as 100 parts by mass, the proportion of the sum of serine, threonine, lysine, and arginine in the amino acids is 10 parts by mass or more, more preferably 15 parts by mass or more, and even more preferably 20 parts by mass or more. Meanwhile, the upper limit of the proportion of the sum of serine, threonine, lysine, and arginine in the amino acids is not particularly limited; however, from the viewpoint that it is difficult to obtain effects appropriate for the content, the upper limit is preferably 70 parts by mass or less.

From the viewpoint that an emulsion having superior stability can be produced, in a case in which the content of PC in the egg yolk phospholipid composition according to the present embodiment is designated as 100 parts by mass, the content of the amino acids with respect to the content of PC is from 0.1 parts by mass to 0.8 parts by mass, and is more preferably 0.2 parts by mass or more, and more preferably 0.7 parts by mass or less.

From the viewpoint of having superior stability, the egg yolk phospholipid composition according to the present embodiment includes PE, and in a case in which the content of PE is designated as 100 parts by mass, the content of the amino acids with respect to the content of PE is from 0.2 parts by mass to 10 parts by mass, and is more preferably 0.5 parts by mass or more, and more preferably 5 parts by mass or less.

Furthermore, in this case, from the viewpoint that an emulsion having superior stability can be produced, in a case in which the content of the egg yolk phospholipids (total amount) in the egg yolk phospholipid composition according to the present embodiment is designated as 100 parts by mass, the content of the amino acids with respect to the content of the egg yolk phospholipids (total amount) is from 0.06 parts by mass to 0.6 parts by mass, and is more preferably 0.1 parts by mass or more, and more preferably 0.5 parts by mass or less.

### (Cholesterol)

In regard to the egg yolk phospholipid composition according to the present embodiment, from the viewpoint that an emulsion having superior stability can be produced, the content of cholesterol (hereinafter, also referred to as "CH") may be 1.5% by mass or less, and the content is preferably 1.0% by mass or less, more preferably 0.8% by mass or less. Meanwhile, the content can be adjusted to 0.01% by mass or more.

According to the present invention, the content of cholesterol is a value measured by the following method.

In a 10-mL stoppered test tube, 0.5 g of a sample is measured and dissolved in chloroform. Subsequently, the solution is diluted to make up 10 mL. This is developed by IATROSCAN TH-10 according to the operation procedure, and measurement is made. Since the sensitivity varies with the lipid composition, for triglycerides and cholesterol, the area values (AREA) are respectively multiplied by the following coefficients, and corrected area values are obtained.
Triglycerides × 0.9
Cholesterol × 0.5

Based on the corrected area value thus obtained, the area percentage of cholesterol is determined, and thus the content of cholesterol is calculated.

### (Free fatty acids)

In regard to the egg yolk phospholipid composition according to the present embodiment, from the viewpoint that a decrease in pH can be suppressed, and an emulsion having superior stability can be produced thereby, the content of free fatty acids may be 1.0% by mass or less, and the content is preferably 0.8% by mass or less.

According to the present invention, free higher fatty acids do not include fatty acids in the form of fatty acid esters that constitute plant oils or phospholipids. Higher fatty acids are, for example, linear or branched saturated or unsaturated fatty acids having 6 to 22 carbon atoms (preferably 12 to 20 carbon atoms). Examples of the higher fatty acids include oleic acid, stearic acid, linoleic acid, palmitic acid, linolenic acid, and myristic acid, and oleic acid is preferred.

According to the invention, the content of free fatty acids is checked by the following method. A sample solution is produced by adding isopropanol to 1.0 g of a sample to dissolve the sample, and precisely adjusting the volume to 25 mL. Furthermore, a standard solution containing palmitic acid at a proportion of 1.0% by mass or 0.8% by mass is produced by dissolving palmitic acid in heptane. Aliquots of 1 mL each are measured and taken into test tubes from the sample solution and the standard solution thus produced, and 5.0 mL of a liquid mixture obtained by mixing isopropanol:heptane:0.5 mol/L sulfuric acid at a ratio of 40:10:1 is added to the test tube using a graduated pipette. The resulting mixtures are shaken for one minute and then are left to stand for 10 minutes. Subsequently, 3 mL of heptane and 3 mL of distilled water are added to the sample solution, and 2 mL of heptane and 4 mL of distilled water are added to the standard solution. The mixtures are tightly sealed, mixed, and left to stand for 15 minutes or longer to undergo layer separation. 3 mL each of the supernatant liquids are precisely measured into stoppered test tubes, and 1 mL of Nile Blue reagent is added to each test tube. Immediately nitrogen gas is blown into the test tubes to purge the test tubes with nitrogen, and then the mixtures are titrated until the liquid turns purple, using a 0.01 mol/L sodium hydroxide solution. When the amount of the sodium hydroxide solution required for the titration of the sample solution is smaller than the amount of the sodium hydroxide solution required for the titration of the standard solution, the content of fatty acids in the sample is said to be 1.0% by mass or 0.8% by mass.

From the viewpoint of having superior stability, the pH of the egg yolk phospholipid composition according to the present embodiment is preferably from 6.5 to 10, and the pH is more preferably 7.0 or higher, and more preferably 9.5 or lower.

### (Operating effects)

The fat emulsion produced by using the egg yolk phospholipid composition according to the present embodiment has a property that the pH does not readily decrease at the time of sterilization. The inventors of the present invention found that a fat emulsion including an egg yolk phospholipid composition having a content of LPE of 1% by mass or less and a content of amino acids of from 50 mg/100 g to 500 mg/100 g does not readily undergo a decrease in pH at the time of sterilization.

It is speculated that a fat emulsion including the egg yolk phospholipid composition according to the present embodiment does not readily undergo a decrease in pH at the time of sterilization, because of the amino acids included in the egg yolk phospholipid composition. More specifically, it is speculated to be because, when the fat emulsion includes the amino acids in the above-specified amount, the amino acids suppress a decrease in the pH of the fat emulsion, and therefore, the emulsion stability of the fat emulsion can be maintained.

### 2. Method for producing egg yolk phospholipid composition (Not covered by the present claims)

A method for producing an egg yolk phospholipid composition according to an embodiment (not covered by the present claims) includes:
a step of mixing a first egg yolk phospholipid composition including LPE at a content of 1% by mass or less and amino acids at a content of from 0 mg/100 g to 250 mg/100 g (hereinafter, also simply referred to as "first egg yolk phospholipid composition"), with a second egg yolk phospholipid composition including LPE at a content of more than 1% by mass and amino acids at a content of from 250 mg/100 g to 700 mg/100 g (hereinafter, also simply referred to as "second egg yolk phospholipid composition"), and thereby obtaining a final egg yolk phospholipid composition including lysophosphatidylethanolamine at a content of 1% by mass or less and amino acids at a content of from 50 mg/100 g to 500 mg/100 g ("egg yolk phospholipid composition according to the above-described embodiment"; hereinafter, also simply referred to as "final egg yolk phospholipid composition").

The final egg yolk phospholipid composition can also be obtained by mixing the first egg yolk phospholipid composition with a first raw material egg yolk phospholipid composition that will be described below.

Furthermore, in addition to the first egg yolk phospholipid composition and the second egg yolk phospholipid composition, the final phospholipid composition can also be obtained by further incorporating an egg yolk phospholipid composition that has been separately treated, or other components such as amino acids, to the extent that does not impair the effects of the invention.

According to the method (not covered by the present claims) for producing an egg yolk phospholipid composition according to the present embodiment, since the method includes a step of mixing the first egg yolk phospholipid composition with the second egg yolk phospholipid composition, and thereby obtaining the final egg yolk phospholipid composition, a final egg yolk phospholipid composition including a trace amount of amino acids (that is, the content of amino acids is from 50 mg/100 g to 500 mg/100 g) and having the content of LPE reduced to 1% by mass or less, the final egg yolk phospholipid composition not readily undergoing a decrease in pH at the time of sterilization, can be produced by a convenient and efficient method.

The first egg yolk phospholipid composition may be a phospholipid composition produced by mixing commercially available amino acids and commercially available egg yolk phospholipids, or may be a phospholipid composition produced by a first resin treatment step that will be described below.

Above all, from the viewpoint that the content of LPE and the content of amino acids have been reduced to be in predetermined ranges, it is preferable that the first egg yolk phospholipid composition is a phospholipid composition produced by the first resin treatment step that will be described below.

Furthermore, the second egg yolk phospholipid composition may be a phospholipid composition produced by mixing commercially available amino acids and commercially available egg yolk phospholipids, may be a phospholipid composition produced by a second resin treatment step that will be described below, or may be a phospholipid composition produced by mixing a phospholipid composition produced by the second resin treatment step that will be described below, with commercially available amino acids and/or commercially available egg yolk phospholipids.

From the viewpoint that the content of LPE and the content of amino acids have been reduced to be in predetermined ranges, it is preferable that the second egg yolk phospholipid composition is a phospholipid composition produced by the second resin treatment step that will be described below.

### (Example of method (not covered by the present claims) for producing egg yolk phospholipid composition according to present embodiment using resin treatment)

An example of the method (not covered by the present claims) for producing an egg yolk phospholipid composition according to the present embodiment includes a first resin treatment step that will be described below, a second resin treatment step that will be described below, and a step of mixing the first egg yolk phospholipid composition with the second egg yolk phospholipid composition, and thus obtaining a final egg yolk phospholipid composition (egg yolk phospholipid composition according to the embodiment described above) (hereinafter, also simply referred to as "step of mixing").

The inventors of the present invention found that a fat emulsion produced by using an egg yolk phospholipid composition having the content of LPE reduced by a resin treatment is prone to have the pH decreased at the time of sterilization, that as a cause of the decreased pH, the amino acids in the egg yolk phospholipid composition are eliminated together with LPE at the time of a resin treatment, and that during the resin treatment, the amino acids tend to be eliminated first before the LPE.

In this regard, when the method (not covered by the present claims) for producing an egg yolk phospholipid composition according to the present example is used, since the method includes the first resin treatment step, the second resin treatment step, and the step of obtaining a final egg yolk phospholipid composition, a final egg yolk phospholipid composition which includes a trace amount of amino acids, has the content of LPE reduced to 1% by mass or less, and does not readily undergo a decrease in pH at the time of sterilization, can be produced by a convenient and efficient method.

That is, a first egg yolk phospholipid composition having a smaller content of amino acids (for example amino acids and LPE) is obtained by the first resin treatment step, and a second egg yolk phospholipid composition having a larger content of amino acids (for example, amino acids and LPE) is obtained by the second resin treatment step. By mixing this first egg yolk phospholipid composition and the second egg yolk phospholipid composition, a final egg yolk phospholipid composition can be produced by a convenient and efficient method.

### (First resin treatment step)

The first resin treatment step is a step of obtaining the first egg yolk phospholipid composition by subjecting a first raw material egg yolk phospholipid composition including LPE at a content of more than 1% by mass and 3% by mass or less and amino acids at a content of more than 500 mg/100 g, to a resin treatment using a first ion exchange resin.

In regard to the first resin treatment step described above and the second resin treatment step that will be described below, the method for resin treatment is not particularly limited; however, for example, the resin treatment may be carried out by mixing the resin with a solution of the raw material egg yolk phospholipid composition in a solvent, or may be carried out by packing a cylindrical-shaped container with the resin, and passing a solution of the raw material egg yolk phospholipid composition through the container.

The time for the first resin treatment is, for example, 1 to 30 minutes, and preferably 1 minute or longer and shorter than 10 minutes. It is preferable that the time for the first resin treatment is shorter than the time for the second resin treatment that will be described below. Furthermore, the temperature for the first resin treatment is, for example, 20°C to 40°C.

According to the first resin treatment step, since the ability to eliminate amino acids (as well as LPE) is high compared to the second resin treatment step that will be described below, a first egg yolk phospholipid composition having reduced amino acids (as well as LPE) can be obtained in a short period of time.

### (Second resin treatment step)

The second resin treatment step is a step of obtaining the second egg yolk phospholipid composition by subjecting a second raw material egg yolk phospholipid composition including LPE at a content of more than 1% by mass and 3% by mass or less and amino acids at a content of more than 500 mg/100 g, to a resin treatment using a second ion exchange resin.

The time for the second resin treatment is, for example, 10 minutes to 1 hour, and preferably 20 minutes or longer and shorter than 40 minutes. Furthermore, the temperature for the second resin treatment is, for example, 20°C to 40°C.

According to the second resin treatment step, since the ability to eliminate amino acids as well as LPE is low compared to the first resin treatment step, a second egg yolk phospholipid composition having a large content of amino acids (as well as LPE) compared to the first egg yolk phospholipid composition can be obtained.

### (Raw material egg yolk phospholipid compositions)

(First and second) raw material egg yolk phospholipid compositions usually include egg yolk phospholipids (total amount) in an amount of from 80% by mass to 99% by mass, and for example, in the raw material egg yolk phospholipid compositions, the percentage content of PC in the egg yolk phospholipids (total amount) is from 60% by mass to 80% by mass, and the percentage content of PE is from 5% by mass to 20% by mass.

Furthermore, the content of LPE in the raw material egg yolk phospholipid compositions is preferably more than 1% by mass and is preferably 3% by mass or less, and the content of amino acids is usually more than 500 mg/100 g. When the contents are within the ranges described above, the same raw material egg yolk phospholipid composition may be used, or different compositions may be used, for the first raw material egg yolk phospholipid composition and the second raw material egg yolk phospholipid composition.

Meanwhile, it is preferable that the raw material egg yolk phospholipid compositions include at least one selected from serine, threonine, lysine, and arginine, and it is more preferable that the raw material egg yolk phospholipid compositions include serine and/or threonine. The amino acids included in the egg yolk phospholipid compositions according to the present embodiment may be amino acids that are originally included in the raw material egg yolk phospholipid compositions, or may be amino acids that have been added to an egg yolk phospholipid composition obtainable by performing a treatment using an ion exchange resin. However, the amino acids can be amino acids that are originally included in the raw material egg yolk phospholipid compositions.

### (Production of raw material egg yolk phospholipid composition)

In regard to the method (not covered by the present claims) for producing an egg yolk phospholipid composition according to the present embodiment, the raw material egg yolk phospholipid composition is a raw material egg yolk phospholipid composition obtained by extracting egg yolk with an alcohol, removing the alcohol, subsequently adding acetone in a volume which is 18 or more times the volume of the egg yolk phospholipids, and then removing the acetone.

The alcohol used for the alcohol extraction is not particularly limited; however, examples include methanol, ethanol, n-propanol, isopropanol, n-butyl alcohol, 2-butyl alcohol, and tert-butyl alcohol. Among these, it is preferable to use a lower alcohol having from one to three carbon atoms. As a result of this alcohol extraction, an alcohol extract of egg yolk is obtained. Furthermore, from the viewpoint of being superior for the LPE removal efficiency, the concentration of the alcohol in the extraction solvent is preferably more than 95% by mass, and more preferably 96% by mass or more.

Furthermore, an egg yolk phospholipid composition having a decreased content of cholesterol can be obtained by removing the alcohol from the alcohol extract of egg yolk, subsequently adding acetone in a volume which is 18 or more times the volume of the raw material egg yolk phospholipid composition, and removing the acetone. In this case, from the viewpoint that the cholesterol content can be further decreased, the amount of acetone to be added is preferably 20 or more times.

### (Ion exchange resin)

Examples of the first and second ion exchange resins used for the first resin treatment step and the second resin treatment step include: strongly acidic or weakly acidic cation exchange resins (AMBERLITE IR120B, IR124, 200CT, 252, FPC3500, and IRC76); and strongly basic or weakly basic anion exchange resins (IRA400J, IRA402BL, IRA404J, IRA458RF, and IRA67). These may be used singly or in combination of a plurality of resins. It is preferable that a cation exchange resin and an anion exchange resin are used in combination, and it is more preferable that a cation exchange resin and an anion exchange resin are used at a proportion of higher than 1/1 (v/v) and 1/3 (v/v) or lower.

### (Rate of decrease in amino acids)

In the first resin treatment step, the rate of decrease in the amount of amino acids (as well as LPE) in the egg yolk phospholipid composition is higher than that in the second resin treatment step. Therefore, the first egg yolk phospholipid composition obtainable by the first resin treatment step has a lower content of amino acids (for example, amino acids and LPE) than the second egg yolk phospholipid composition obtainable by the second resin treatment composition. That is, the first egg yolk phospholipid composition having a lower content of amino acids (for example, amino acids and LPE) than the second egg yolk phospholipid composition can be obtained by the first resin treatment step.

More specifically, in regard to the first resin treatment step, the rate of decrease in the content of amino acids in the egg yolk phospholipid composition per minute is preferably from 100 mg/100 g to 250 mg/100 g, and the rate is more preferably 150 mg/100 g or more and more preferably 200 mg/100 g or less.

Furthermore, in regard to the second resin treatment step, the rate of decrease in the content of amino acids in the egg yolk phospholipid composition per minute is from 3 mg/100 g to 30 mg/100 g, and the rate is more preferably 5 mg/100 g or more and more preferably 25 mg/100 g or less.

It is preferable to adjust the type and amount of the ion exchange resin such that, for example, the rates of decrease in the amount of amino acids (as well as LPE) in the first resin treatment step and the second resin treatment step are in the above-mentioned range, so that the first egg yolk phospholipid composition and the second egg yolk phospholipid composition are obtained.

In regard to the first resin treatment step and the second resin treatment step, in a case in which a raw material egg yolk phospholipid composition is treated using an ion exchange resin, for example, a method of dissolving the raw material egg yolk phospholipids in a polar solvent (for example, an alcohol such as methanol or ethanol, or water) in an amount of 1 to 5 times the amount of the raw material egg yolk phospholipid composition, or a mixed solvent of the above-mentioned polar solvent and a solvent other than the polar solvent (for example, a halogen-based solvent such as dichloromethane or chloroform; a ketone-based solvent such as acetone or methyl ethyl ketone; an ester-based solvent such as ethyl acetate; a hydrocarbon-based solvent such as n-hexane or n-pentane; or an ether-based solvent such as diethyl ether), bringing the solution into contact with an ion exchange resin, and then distilling off the solvent under reduced pressure, may be mentioned. In this case, the polar solvents and the mixed solvents can be used singly or in combination of a plurality of the solvents.

An ion exchange resin is a synthetic resin having a structure in which a functional group such as a sulfonic acid group or a quaternary ammonium group has been introduced into a crosslinked three-dimensional polymer parent body. In order to perform an efficient resin treatment, generally, the resin is used after the specific surface of the resin is enlarged by hydrating the resin itself with water or the like and thereby forming voids in the structure.

Meanwhile, in a case in which the raw material egg yolk phospholipid composition is treated using an ion exchange resin, the raw material egg yolk phospholipid composition is dissolved in a polar solvent or a mixed solvent as described above. When the raw material egg yolk phospholipid composition is exposed to such as polar solvent or a mixed solvent, it is empirically known that the ion exchange resin is dehydrated over time.

When the ion exchange resin is dehydrated, the voids existing at the time of hydration shrink or open up. Particularly, a component having a relatively large molecular weight, such as LPE, cannot efficiently come into contact with the functional groups existing in the voids of the ion exchange resin, and the ability to eliminate LPE is deteriorated. Therefore, in a case in which a component having a relatively large molecular weight, such as LPE, is taken as the object of treatment under the conditions in which the ion exchange resin is dehydrated over time, it is desirable to use an ion exchange resin in a state in which a sufficient amount of voids exist, that is, an ion exchange resin having a larger volume.

Furthermore, the volume of the ion exchange resin is generally dependent on the type of the ion that forms a pair with the functional group. Specifically, in the case of an anion exchange resin, as the quantity of Cl⁻ is larger than the quantity of OH⁻, the volume tends to become smaller.

In view of the matters described above, in the first resin step, from the viewpoint that it is preferable to use an ion exchange resin having a larger volume in order to enhance the ability to eliminate LPE, the anion exchange resin used for the first resin treatment step is preferably an anion exchange resin in which the proportion of Cl⁻ in the anions that bind to the functional groups is 0.2 equivalent% or less. Meanwhile, in the second resin step, from the viewpoint that it is preferable to use an ion exchange resin having a smaller volume than that used for the first resin treatment step in order to control the LPE removal efficiency, the anion exchange resin used for the second resin treatment step is preferably an anion exchange resin in which the proportion of Cl⁻ in the anions that bind to the functional groups is more than 0.2 equivalent%.

In regard to the first resin treatment step and the second resin treatment step, alumina can also be used in place of the ion exchange resin, or together with the ion exchange resin.

### (Step of mixing)

The step of mixing is a step of mixing the first egg yolk phospholipid composition with the second egg yolk phospholipid composition, and thereby obtaining the final egg yolk phospholipid composition (egg yolk phospholipid composition according to the embodiment described above).

In the step of mixing, from the viewpoint that the egg yolk phospholipid composition according to the embodiment described above (final egg yolk phospholipid composition) can be easily obtained, the ratio of first egg yolk phospholipid composition to second egg yolk phospholipid composition (mass ratio) is usually from 0.2 to 5, and the ratio is preferably 0.5 or higher and is preferably 4 or lower.

### 3. Fat emulsion

A fat emulsion according to an embodiment of the present invention consists of: emulsified particles consisting of the egg yolk phospholipid composition according to the embodiment described above; and an aqueous phase in which the emulsified particles are dispersed.

Since the fat emulsion according to the present embodiment consists of: emulsified particles consisting of the egg yolk phospholipid composition according to the embodiment described above; and an aqueous phase in which the emulsified particles are dispersed, the fat emulsion has high purity and excellent emulsion stability.

### (Average particle size)

In regard to the fat emulsion according to the present embodiment, the average particle size of the emulsified particles is from 0.1 µm to 0.5 µm, and from the viewpoint of having superior stability, the average particle size is preferably 0.3 µm or less.

### (Oil phase)

In regard to the fat emulsion according to the present embodiment, examples of the oil phase that constitutes the emulsified particles include: plant oils such as soybean oil, sesame oil, rapeseed oil, safflower oil, olive oil, castor oil, corn oil, cotton seed oil, rice oil, sunflower oil, grape seed oil, and wheat germ oil; and medium-chain fatty acid triglycerides (MCT). The plant oil is preferably purified plant oil.

### (Composition of fat emulsion)

It is preferable that the fat emulsion according to the present embodiment is, for example, an emulsion having from 65% by mass to 90% by mass of an aqueous phase, and from 10% by mass to 30% by mass of an oil phase.

### (Production of fat emulsion)

For example, egg yolk phospholipids and glycerin as optional additive are mixed and homogenized with a predetermined amount of an oil phase using a commonly used homogenizer. Subsequently, a necessary amount of water is added to this homogenization product, and then homogenization is carried out again with the homogenizer to convert the homogenization product into an oil-in-water type emulsion (emulsification product). Thus, the fat emulsion according to the present embodiment can be produced.

### (Lipoid preparation)

A lipoid preparation according to the embodiment described above consists of: emulsified particles consisting of the egg yolk phospholipid composition according to the embodiment described above and a drug; and an aqueous phase in which the emulsified particles are dispersed, and the lipoid preparation has high purity and excellent emulsion stability.

In regard to the lipoid preparation according to the present embodiment, the average particle size of the emulsified particles is from 0.1 µm to 0.5 µm, and from the viewpoint of having superior stability, the average particle size is preferably 0.3 µm or less.

Regarding the oil phase that constitutes the lipoid preparation according to the present embodiment, for example, those mentioned as examples as the oil phase that constitutes the emulsified particles according to the embodiment described above can be used.

The drug that constitutes the lipoid emulsion according to the present embodiment is not limited; however, for example, an oleophilic drug is preferable. Examples of drugs that are currently available in the market include prostaglandin (PGE1), steroids (dexamethasone palmitate), NSAIDS (flurbiprofen axetil), and propofol. In addition to these, more specific examples include diazepam, clevidipine butyrate, and indomethacin.

The lipoid preparation according to the present embodiment is preferably an emulsion having, for example, from 65% by mass to 90% by mass of an aqueous phase and from 10% by mass to 30% by mass of an oil phase, and the content of the drug is, for example, from 5 µg/mL to 10 % by mass.

### (Production of lipoid preparation)

For example, egg yolk phospholipids, a drug and glycerin as optional additive are mixed and homogenized with a predetermined amount of an oil phase using a commonly used homogenizer, and then a necessary amount of water is added to this homogenization product. Subsequently, homogenization is performed again with the homogenizer to convert the homogenization product into an oil-in-water type emulsion (emulsification product). Thus, the lipoid preparation according to the present embodiment can be produced.

### Examples

### 4. Examples

Hereinafter, the present invention will be described in more detail by way of Examples; however, the present invention is not intended to be limited to the Examples.

### [Example 1]

Dried egg yolk was subjected to alcohol (96 mass% ethanol-containing ethanol-water liquid mixture) extraction, subsequently the solvent was removed under reduced pressure, and the residue was treated with acetone in a 20-fold amount. Thus, a raw material egg yolk phospholipid composition having the following composition (first raw material egg yolk phospholipid composition and second raw material egg yolk phospholipid composition) was obtained.

| | |
|---|---|
| PC | 75 % by mass |
| PE | 18 % by mass |
| LPE | 2.5 % by mass |
| CH | 0.8 % by mass |
| Amino acids | 0.6 % by mass |

100 g of the raw material egg yolk phospholipid composition was dissolved in 300 ml of a solvent (hexane-methanol (80:20), then the solution was introduced into a container containing a first ion exchange resin (12 mL of product name: AMBERLITE IR120B (cation exchange resin: H type) and 24 mL of AMBERLITE IRA400 (anion exchange resin: OH type)), and the mixture was stirred. Thus, a resin treatment (temperature: 20°C to 40°C, time: for 4 minutes) was carried out. Thereby, a first egg yolk phospholipid composition including LPE at a content of 0.5% by mass and amino acids at a content of 10 mg/100 g was obtained (first resin treatment step). In the first resin treatment step, the rate of decrease in the content of amino acids in the egg yolk phospholipid composition per minute was 150 mg/100 g.

100 g of the raw material egg yolk phospholipid composition was dissolved in 300 ml of a solvent (hexane-methanol (80:20), then the solution was introduced into a container containing a second ion exchange resin (6 mL of product name: AMBERLITE IR120B (cation exchange resin: H type) and 10 mL of AMBERLITE IRA404 (anion exchange resin: OH type)), and the mixture was stirred. Thus, a resin treatment (temperature: 20°C to 40°C, time: for 20 minutes) was carried out. Thereby, a second egg yolk phospholipid composition including LPE at a content of 1.5% by mass and amino acids at a content of 400 mg/100 g was obtained (second resin treatment step). In the second resin treatment step, the rate of decrease in the content of amino acids in the egg yolk phospholipid composition per minute was 10 mg/100 g.

Next, the first egg yolk phospholipid composition and the second egg yolk phospholipid composition were mixed so as to obtain a ratio of first egg yolk phospholipid composition to second egg yolk phospholipid composition (mass ratio) of 1/1. Thus, the final egg yolk phospholipid composition of Example 1 having the following composition was obtained. The content of free fatty acids in the egg yolk phospholipid composition thus obtained was 0.2% by mass.

| | |
|---|---|
| PC | 75 % by mass |
| PE | 18 % by mass |
| LPE | 1.0 % by mass |
| CH | 0.8 % by mass |
| Amino acids | 0.4 % by mass |

### [Example 2]

In regard to the second resin treatment step of Example 1, a treatment similar to that performed in Example 1 was carried out, except that 100 g of alumina (product name: activated alumina (manufactured by Wako Pure Chemical Industries, Ltd.) was used instead of an ion exchange resin. Thus, an egg yolk phospholipid composition of Example 2 having the following composition was obtained. Meanwhile, the content of free fatty acids in the egg yolk phospholipid composition thus obtained was 0.2 % by mass.

| | |
|---|---|
| PC | 80 % by mass |
| PE | 8 % by mass |
| LPE | 0.5 % by mass |
| CH | 0.8 % by mass |
| Amino acids | 0.2 % by mass |

### [Test Example 1]

In regard to Example 1 described above, five kinds of egg yolk phospholipid compositions (Test Nos. 1 to 5; Test No. 1 is not covered by the present invention) having different amino acid contents were produced by varying the amount of resins and the reaction time (Table 1). For each of the phospholipids, a fat emulsion was produced using a high-pressure homogenizer.

The pH of each of the fat emulsions of Test Example 1 was from 6.5 to 10, and the average particle size was from 0.1 µm to 0.5 µm. In the present Example, a particle size distribution analyzer manufactured by Beckman Coulter, Inc. (model name: N4 PLUS) was used for the measurement of the average particle size.

These samples were adjusted to pH 9.5 with an aqueous solution of sodium hydroxide and were sterilized at 121°C for 20 minutes. Then, the zeta potential and pH were measured. The results are presented in Table 1.

In the present Example, the zeta potential of the fat emulsion was measured using ZETASIZER NANO Z manufactured by Malvern Panalytical, Ltd., and the pH of the fat emulsion was measured using a pH meter manufactured by Horiba, Ltd. (model name: F-52).

**[Table 1]**

| Test No. | 1 | 2 | 3 | 4 | 5 (Example 1) | Example 2 |
|---|---|---|---|---|---|---|
| Amino acid content (mg/100 g) | 0 | 50 | 100 | 200 | 400 | 200 |
| Zeta potential (mV) | -40 | -46 | -55 | -56 | -57 | -51 |
| Decrease in pH | 2.6 | 2.5 | 2 | 1.7 | 1.3 | 1.4 |

According to Table 1, it can be understood that fat emulsions that exhibit a small decrease in pH (specifically, a decrease of 2.5 or less) and have excellent stability can be obtained by using the egg yolk phospholipid compositions (Test Nos. 2 to 5) according to the present Examples.

Furthermore, Test Nos. 2 to 5 maintain low zeta potentials, and therefore, it is speculated that the decrease in pH at the time of heating and sterilization is small.

In addition, it can be understood from Example 2 that similar effects are obtained even in the case of using alumina.

### [Test Example 2]

To the egg yolk phospholipid composition of Test No. 1 (not covered by the present invention) produced in Test Example 1 described above, serine (Ser), threonine (Thr), valine, leucine, and isoleucine were added, subsequently the mixture was freeze-dried, and fat emulsions were produced from the freeze-dried product using an ultrasonic homogenizer (Test Nos. 6 to 8; Test No. 6 is not covered by the present invention). These samples were adjusted to pH 9.5 using an aqueous solution of sodium hydroxide and were sterilized at 121°C for 20 minutes, and then the pH was measured. The results are presented in Table 2. The pH of the various fat emulsions of Test Example 2 was from 6.5 to 10, and the average particle size was from 0.1 µm to 0.5 µm.

**[Table 2]**

| Test No. | 6 | 7 | 8 |
|---|---|---|---|
| Amino acid content (mg/100 g) | 400 | 400 | 400 |
| Ser and Thr content (mg/100 g) | 0 | 40 | 120 |
| Proportion of Ser and Thr in all amino acids (% by mass) | - | 10 | 30 |
| Decrease in pH | 2.4 | 2.2 | 2 |

According to Table 2, it can be understood that when the proportion of the sum of serine and threonine in the amino acids is 10 parts by mass or more in a case in which the total amount of the amino acids is designated as 100 parts by mass, a fat emulsion that exhibits a small decrease in pH after heating and sterilization and has excellent stability can be easily obtained.

### [Test Example 3]

To Sample 1 produced in Test Example 1 described above, serine (Ser), threonine (Thr), arginine (Arg), lysine (Lys), valine, leucine, and isoleucine were added, and then the mixture was freeze-dried. A fat emulsion was produced using an ultrasonic homogenizer. This sample was adjusted to pH 9.5 with an aqueous solution of sodium hydroxide and was sterilized at 121°C for 20 minutes. Thus, the pH was measured. The results are presented in Table 3 (Test No. 9 - 12; Test. No. 9 is not covered by the present invention). The pH of the various fat emulsions of Test Example 3 was from 6.5 to 10, and the average particle size was from 0.1 µm to 0.5 µm.

**[Table 3]**

| Test No. | 9 | 10 | 11 | 12 |
|---|---|---|---|---|
| Amino acid content (mg/100 g) | 400 | 400 | 400 | 400 |
| Ser, Thr, Arg, and Lys content (mg/100 g) | 0 | 40 | 80 | 240 |
| Proportion of above four kinds in all amino acids (% by mass) | - | 10 | 20 | 60 |
| Decrease in pH | 2.4 | 2 | 2 | 1.8 |

According to Table 3, it can be understood that when the proportion of the sum of serine, threonine, arginine, and lysine in the amino acids is 10 parts by mass or more in a case in which the total amount of the amino acids is designated as 100 parts by mass, a fat emulsion that exhibits a smaller decrease in pH after heating and sterilization and has excellent stability can be easily obtained.

### [Test Example 4]

6 g of the egg yolk phospholipid composition of Example 1 and 100 g of soybean oil were dispersed by stirring the mixture at a high speed, and 384 g of distilled water and 10 g of glycerin were added to the dispersion. Subsequently, crude emulsification (stirring speed: 10,000 rpm, stirring time: for 40 minutes) was carried out, and then fine emulsification (high-pressure emulsification: 500 kg/cm², the liquid was passed repeatedly for 10 to 20 times) was carried out. Subsequently, pH adjusting and filtration were carried out, and then a glass vial was filled with the resultant emulsion. Thus, a fat emulsion of Test Example 4 (20 mass% soybean oil-containing O/W emulsion) was obtained.

The fat emulsion thus obtained was sterilized at 121°C for 20 minutes, and then the bottle was opened. The average particle size and pH of the fat emulsion were measured. The fat emulsion of Test Example 4 underwent a small change in the average particle size before and after sterilization and had high stability, in which the decrease in pH after sterilization was well suppressed.

### [Test Example 5]

6 g of the egg yolk phospholipid composition of Example 1, 50 g of soybean oil, and 10 g of a drug (indomethacin) were dispersed by stirring the mixture at a high speed, and 424 g of distilled water and 10 g of glycerin were added to the dispersion. Subsequently, crude emulsification (stirring speed: 10,000 rpm, stirring time: for 40 minutes) was carried out, and then fine emulsification (high-pressure emulsification: 500 kg/cm², the liquid was passed repeatedly for 10 to 20 times) was carried out. Subsequently, pH adjustment and filtration were carried out, and then a glass vial was filled with the resultant emulsion. Thus, a lipoid preparation of Test Example 5 (10 mass% soybean oil-containing O/W emulsion) was obtained.

The lipoid preparation thus obtained was sterilized at 121°C for 20 minutes, and then the bottle was opened. The average particle size and pH of the lipoid preparation were measured. The lipoid preparation of Test Example 5 underwent a small change in the average particle size before and after sterilization and had high stability, in which the decrease in pH after sterilization was well suppressed.

## Claims

1. An egg yolk phospholipid composition, comprising:
lysophosphatidylethanolamine at a content of 1% by mass or less; amino acids at a content of from 50 mg/100 g to 500 mg/100 g; phosphatidylcholine at a proportion of 65% by mass or more; and phosphatidylethanolamine at a proportion of 5% by mass or more,
wherein in a case in which a total amount of the amino acids is designated as 100 parts by mass, a proportion of a sum of serine, threonine, lysine, and arginine in the amino acids is 10 parts by mass or more,
wherein in a case in which a total amount of egg yolk phospholipids is designated as 100 parts by mass, a content of the amino acids with respect to a content of the egg yolk phospholipids is from 0.06 parts by mass to 0.6 parts by mass,
wherein in a case in which a content of the phosphatidylcholine is designated as 100 parts by mass, a content of the amino acids with respect to the content of the phosphatidylcholine is from 0.1 parts by mass to 0.8 parts by mass,
wherein in a case in which a content of the phosphatidylethanolamine is designated as 100 parts by mass, a content of the amino acids with respect to the content of the phosphatidylethanolamine is from 0.2 parts by mass to 10 parts by mass.

2. The egg yolk phospholipid composition according to claim 1,
wherein in a case in which a content of the phosphatidylethanolamine is designated as 100 parts by mass, a content of the lysophosphatidylethanolamine with respect to the content of the phosphatidylethanolamine is from 3 parts by mass to 15 parts by mass.

3. The egg yolk phospholipid composition according to claim 1 or 2, further comprising cholesterol at a content of 1.5% by mass or less.

4. The egg yolk phospholipid composition according to any one of claims 1 to 3, further comprising free fatty acids at a content of 1.0% by mass or less.

5. A fat emulsion consisting of (a) and (b) below:
(a) emulsified particles consisting of an oil phase and the egg yolk phospholipid composition according to any one of claims 1 to 4; or emulsified particles consisting of an oil phase, the egg yolk phospholipid composition according to any one of claims 1 to 4, and glycerin, and
(b) an aqueous phase in which the emulsified particles are dispersed,
wherein the average particle size of the emulsified particles is from 0.1 µm to 0.5 µm.

6. A lipoid preparation consisting of (a) and (b) below:
(a) emulsified particles consisting of an oil phase and the egg yolk phospholipid composition according to any one of claims 1 to 4 and a drug; or
emulsified particles consisting of an oil phase, the egg yolk phospholipid composition according to any one of claims 1 to 4, a drug and glycerin, and
(b) an aqueous phase in which the emulsified particles are dispersed,
wherein the average particle size of the emulsified particles is from 0.1 µm to 0.5 µm.

## Patentansprüche

1. Eigelbphospholipidzusammensetzung, umfassend:
Lysophosphatidylethanolamin mit einem Gehalt von 1 Massenprozent oder weniger; Aminosäuren mit einem Gehalt von 50 mg/100 g bis 500 mg/100 g; Phosphatidylcholin mit einem Anteil von 65 Massenprozent oder mehr; und Phosphatidylethanolamin mit einem Anteil von 5 Massenprozent oder mehr,
wobei in einem Fall, in dem eine Gesamtmenge der Aminosäuren als 100 Masseteile bezeichnet wird, ein Anteil einer Summe von Serin, Threonin, Lysin und Arginin in den Aminosäuren 10 Masseteile oder mehr beträgt,
wobei in einem Fall, in dem eine Gesamtmenge an Eigelbphospholipiden als 100 Masseteile bezeichnet wird, ein Gehalt der Aminosäuren in Bezug auf einen Gehalt der Eigelbphospholipide von 0,06 Masseteile bis 0,6 Masseteile beträgt,
wobei in einem Fall, in dem ein Gehalt des Phosphatidylcholins als 100 Masseteile bezeichnet wird, ein Gehalt der Aminosäuren in Bezug auf den Gehalt des Phosphatidylcholins von 0,1 Masseteile bis 0,8 Masseteile beträgt,
wobei in einem Fall, in dem ein Gehalt des Phosphatidylethanolamins als 100 Masseteilen bezeichnet wird, ein Gehalt der Aminosäuren in Bezug auf den Gehalt des Phosphatidylethanolamin von 0,2 Masseteile bis 10 Masseteile beträgt.

2. Eigelbphospholipidzusammensetzung nach Anspruch 1,
wobei in einem Fall, in dem ein Gehalt des Phosphatidylethanolamins als 100 Masseteile bezeichnet wird, ein Gehalt des Lysophosphatidylethanolamins in Bezug auf den Gehalt des Phosphatidylethanolamins von 3 Masseteile bis 15 Masseteile beträgt.

3. Eigelbphospholipidzusammensetzung nach Anspruch 1 oder 2, die ferner Cholesterin bei einem Gehalt von 1,5 Massenprozent oder weniger umfasst.

4. Eigelbphospholipidzusammensetzung nach einem der Ansprüche 1 bis 3, die ferner freie Fettsäuren bei einem Gehalt von 1,0 Massenprozent oder weniger umfasst.

5. Fettemulsion, bestehend aus nachfolgendem (a) und (b):
(a) emulgierte Partikel, bestehend aus einer Ölphase und der Eigelbphospholipidzusammensetzung gemäß einem der Ansprüche 1 bis 4; oder
emulgierte Partikel, bestehend aus einer Ölphase, der Eigelbphospholipidzusammensetzung gemäß einem der Ansprüche 1 bis 4 und Glycerin, und
(b) eine wässrige Phase, in der die emulgierten Partikel dispergiert sind,
wobei die mittlere Partikelgröße der emulgierten Partikel von 0,1 µm bis 0,5 µm beträgt.

6. Lipoidpräparat, bestehend aus nachfolgendem (a) und (b):
(a) emulgierte Partikel, bestehend aus einer Ölphase und der Eigelbphospholipidzusammensetzung gemäß einem der Ansprüche 1 bis 4 und einem Medikament; oder
emulgierte Partikel, bestehend aus einer Ölphase, der Eigelbphospholipidzusammensetzung gemäß einem der Ansprüche 1 bis 4, einem Medikament und Glycerin, und
(b) eine wässrige Phase, in der die emulgierten Partikel dispergiert sind,
wobei die mittlere Partikelgröße der emulgierten Partikel von 0,1 µm bis 0,5 µm beträgt.

## Revendications

1. Composition de phospholipides de jaune d'oeuf, comprenant :
de la lysophosphatidyléthanolamine à une teneur de 1 % en masse ou moins ; des acides aminés à une teneur de 50 mg/100 g à 500 mg/100 g ; de la phosphatidylcholine à raison de 65 % en masse ou plus ; et de la phosphatidyléthanolamine à raison de 5 % en masse ou plus,
dans laquelle, dans un cas où une quantité totale des acides aminés est désignée comme étant de 100 parties en masse, une proportion d'une somme de sérine, thréonine, lysine et arginine dans les acides aminés est de 10 parties en masse ou plus,
dans laquelle, dans un cas où une quantité totale de phospholipides de jaune d'oeuf est désignée comme étant de 100 parties en masse, une teneur en acides aminés par rapport à une teneur en phospholipides de jaune d'oeuf est de 0,06 partie en masse à 0,6 partie en masse,
dans laquelle, dans un cas où une teneur en phosphatidylcholine est désignée comme étant de 100 parties en masse, une teneur en acides aminés par rapport à la teneur en phosphatidylcholine est de 0,1 partie en masse à 0,8 partie en masse,
dans laquelle, dans un cas où une teneur en phosphatidyléthanolamine est désignée comme étant de 100 parties en masse, une teneur en acides aminés par rapport à la teneur en phosphatidyléthanolamine est de 0,2 partie en masse à 10 parties en masse.

2. Composition de phospholipides de jaune d'oeuf selon la revendication 1,
dans laquelle, dans un cas où une teneur en phosphatidyléthanolamine est désignée comme étant de 100 parties en masse, une teneur en lysophosphatidyléthanolamine par rapport à la teneur en phosphatidyléthanolamine est de 3 parties en masse à 15 parties en masse.

3. Composition de phospholipides de jaune d'oeuf selon la revendication 1 ou 2, comprenant en outre du cholestérol à une teneur de 1,5 % en masse ou moins.

4. Composition de phospholipides de jaune d'oeuf selon l'une quelconque des revendications 1 à 3, comprenant en outre des acides gras libres à une teneur de 1,0 % en masse ou moins.

5. Émulsion grasse consistant en (a) et (b) ci-dessous :
(a) des particules émulsionnées consistant en une phase huileuse et la composition de phospholipides de jaune d'oeuf selon l'une quelconque des revendications 1 à 4 ; ou
des particules émulsionnées consistant en une phase huileuse, la composition de phospholipides de jaune d'oeuf selon l'une quelconque des revendications 1 à 4, et de la glycérine, et
(b) une phase aqueuse dans laquelle les particules émulsionnées sont dispersées,
dans laquelle la taille de particule moyenne des particules émulsionnées est comprise entre 0,1 µm et 0,5 µm.

6. Préparation lipoïde consistant en (a) et (b) ci-dessous :
(a) des particules émulsionnées consistant en une phase huileuse et la composition de phospholipides de jaune d'oeuf selon l'une quelconque des revendications 1 à 4 et un médicament ; ou
des particules émulsionnées consistant en une phase huileuse, la composition de phospholipides de jaune d'oeuf selon l'une quelconque des revendications 1 à 4, un médicament et de la glycérine, et
(b) une phase aqueuse dans laquelle les particules émulsionnées sont dispersées,
dans laquelle la taille de particule moyenne des particules émulsionnées est comprise entre 0,1 µm et 0,5 µm.
